# EUROPÄISCHE PATENTANMELDUNG

(11) **EP 1 955 734 A1**
(43) Veröffentlichungstag der Anmeldung: **13.08.2008**
(21) Anmeldenummer: 07002476.5
(22) Anmeldetag: 06.02.2007
(51) Int. Cl.: A61N 5/10, A61B 6/04

(54) **Vorrichtung zur Positionierung eines atmenden Menschen**

(71) Anmelder: Siemens Schweiz AG, 8047 Zürich (CH)
(72) Erfinder: Broennimann, Thomas, 4704 Wolfisberg (DE); Cloutot, Laurent, 8956 Killwangen (CH)
(74) Vertreter: Fischer, Michael

(57) **Zusammenfassung**

Es wird eine Vorrichtung zur Positionierung eines atmenden Menschen beschrieben, welcher auf einem Tisch z.B. während einer radiotherapeutischen Bestrahlung liegt. Dabei umfasst der Tisch einen entlang des Tisches zweidimensionalen Bereich welcher zumindest entlang einer dem Tisch senkrechten, dritten Dimension verformbar ist und mit welchem während Atemzyklen des Menschen ein Oberkörperteil des Menschen entlang der dritten Dimension in einem im Koordinatensystem des Tisches fest gezielten Volumen positionierbar ist, als mindestens ein weiterer Oberkörperteil des Menschen durch Verformung des Bereiches frei positionierbar ist. Es werden mehrere Vorteile abgeleitet, insbesondere in Gebiet der Radio-Onkologie in Sinne von Reproduzierbarkeit, Genauigkeit, Schnelligkeit, Sicherheit und Komfort des Patienten.

## Beschreibung

Die Erfindung betrifft eine Vorrichtung zur Positionierung eines atmenden Menschen und Verwendungen der Vorrichtung nach dem Oberbegriff des Anspruches 1 bzw. der korrespondierenden Verwendungsansprüche 24, 25 und 29.

Die auf ungesunde organische Zellen wie einen Tumor zielorientierte Bestrahlung leidet durch eine unzuverlässige (da nicht ersichtliche) räumliche Verfolgung des bewegbaren Tumors im Thorax-Bereich aufgrund der Atmung des auf dem Tisch liegenden Patienten während einer radio-onkologischen Behandlung. Dies bedeutet:
- häufige Ein- und Ausschalten des Bestrahlungseinrichtung sind notwendig, (nur Einschaltung, wenn der Tumor in einem "geschätzten" Referenzbereich sich befindet);
- zu hohe Behandlungsdauer sind notwendig für den Patient, der trotzdem möglichst sehr ruhig bleiben muss;
- eine sehr breite Bestrahlungszone wird aus "Sicherheitsgründen" für eine Bestrahlung des vollständigen Tumors benötigt, durch welche andere dem Tumor benachbarte Organe oder gesunde Zellen beschädigen werden können, sowie eine unnötige schädliche, akkumulierte Energiemenge für den meistens bereits sehr schwachen Patient ist zur Zeit schwierig zu vermeiden.
   Eine erste Lösung besteht darin, einstellbare Strahlenmechanismen zur variablen, möglichst kontinuierlichen Erzielung des bewegbaren Tumors (gesteuerter Roboter/beweglicher Arm, mechanische oder elektromagnetische Strahlablenkung, verformbare Blenden, etc) zu verwenden. Nun sind diese mechanischen Methoden für feinere Bewegungen wie für diejenigen aus dem Atem nicht genau geeignet. Sie sind allerdings aufwendig, kompliziert zu steuern und daher auch sehr teuer aufgrund ihrer Komplexität.
   Als Alternativ zu diesen letzten Methoden ist gemäß bzw. in Synchronisation mit der Atembedingten Bewegung des Tumors ein Hin- und Herfahren des Liegetisches relativ zur Bestrahlungseinrichtung möglich, jedoch ist eine derartige Bewegung des ganzen Patienten nicht bequem da wegen dem zyklischen, z.B. Unten- und Obenfahren des Liegetisches es einem bereits schwachen Patienten schnell schwindelig wäre.
   Ein weiterer Gesichtspunkt ist der geringe "Komfort" für den Patient, da der Tisch im Wesentlichen eine flache Ebeneplatte ist, die bis heute eine räumliche Referenzposition des Patienten bildet. Aufgrund von Mängeln an Komfort wird der Patient höchstwahrscheinlich während einer Bestrahlungsphase von z.B. 10 Minuten seine Position leicht ändern, was zu einer weiteren Ungenauigkeit in der Verfolgung des Tumors unvermeidlich führt.
   Eine weitere Problematik liegt auch daran, dass Fixierungs- bzw. Stabilisierungsmitteln des Patienten gegenüber einer Referenzstellung oft verwendet wird. Dafür wird z.B. der Patient in einer Vakuumhülle gewickelt, so dass zumindest im Thoraxbereich räumliche Abweichungen minimiert werden. Andere Befestigungs-oder Anhaltungsmitteln an dem Tisch sind auch vorstellbar.
   Nun bleiben alle diese Methoden Hilfsmitteln, welche ungenau, aufwendig und teilweise unbequem für den Patient sind.
   Heutzutage wird auch zur genauen Erfassung des Tumors eine Ionisierungsanlage verwendet, welche auch bei der Strahlung des Tumors auf ein zusätzliches Ionisierungsverfahren basiert. Die Ionisierungsanlage weist einen Strahler zur Ionisierungsstrahlung über eine Seitenfläche des Thorax und einen plattenförmigen Silicium-Empfänger hinter der entgegen gesetzten Seitenfläche des Thorax auf. Mit dem Empfänger ist der Tumor leicht zu erkennen bzw. zu erfassen. Aus dem Empfänger werden zwei dimensionale Koordinaten des Tumors z.B. im Koordinatensystem des Tisches oder eines Isozentrum der Bestrahlungsanlage für den Tumor berechnet. Bewegt man die Ionisierungsanlage von einem definierten und bekannten Winkel zu dem Patient, werden zwei neue, auch zwei dimensionale Koordinaten ermittelt, aus welchen in Verbindung mit den ersten zwei dimensionalen Koordinaten des Tumors die drei dimensionalen Koordinaten des Tumors im Koordinatensystem errechnet werden. Für eine bessere Genauigkeit der Ermittlung der Tumorposition sollten beide Aufnahmen z.B. bei Vollatmung des Patienten durchgeführt werden. Hier stellt sich noch leider die Frage, ob die Reproduzierbarkeit der Lage des Tumors bei unterschiedlichen Vollatmungen gesichert ist. Weiterhin ist ein derartiges Ionisierungsverfahren nicht unschädlich wegen der Energiemenge an Strahlung. Dies bedeutet, dass schwer erkrankte Patienten selten bzw. nicht mehr mit einem solchen Verfahren behandelt werden.
   Es wird auch vor einer Bestrahlungsbehandlung oder regelmäßig während der Bestrahlungsperiode (z.B. von 4-6 Wochen) eine Computertomographie CT durchgeführt, bei welcher zumindest zwei Positionen des Tumors bei Vollein- und ausatmen ermittelt werden. Damit wird besser mittels eines therapeutischen Planungstools TPS abgeschätzt, in welchem Volumenbereich des Thorax eine hoch energetische (z.B. Gamma-, Protonen-, etc.) Strahlung bei einer anschließenden Bestrahlungsbehandlung erfolgen muss. Dieser volumenbereich wird praktisch individuell für den Patient gespeichert und bei der regelmäßigen Positionierung des Patienten auf dem Liegetisch wird angestrebt, dass räumliche Abweichungen zu den gespeicherten Daten nicht vorliegen. Hier besteht immer noch eine Ungenauigkeit in Sinne des Reproduzierbarkeit zwischen zeitlich entfernten Neupositionierungen des Patienten gegenüber der Bestrahlungsanlage gegen einen Tumor.
   Weitere Positionierungshilfsmitteln wie vordefinierte geplante Bewegungssimulationen, mechanische Umlenkelemente, ein Atemmessender Brustgurt, photogrammetrische Markierungen, optische Abbildungsverfahren (z.B. 2- oder 3-dimensionale Erfassung des Brustkorbs) können auch verwendet werden, jedoch weisen immer eine kritische Unsicherheit dabei auf, und sind meistens in Sinne einer guten Reproduzierbarkeit noch nicht optimal. Ferner ist es üblich, dass während der gesamten Bestrahlungstherapie Patienten abnehmen, so dass ein Gewichtsfaktor die Reproduzierbarkeit auch noch erschweren kann.
   Der Erfindung liegt die Aufgabe zugrunde, eine Vorrichtung zur Positionierung eines atmenden, auf einem Tisch liegenden Menschen, bei welcher eine genauere Positionierung eines Oberköperteils des Menschen, wie eines Tumors oder eines Karzinoms, in einem bekannten Koordinatensystem gewährleistet wird.
   Es sollten auch Verwendungen dieser Vorrichtung gewährleistet werden, welche vorteilhafte Verbesserung in Sinne von Positionierung, Komfort, Reproduzierbarkeit, Sicherheit des Patienten ermöglichen.
   Erfindungsgemäß wird die Aufgabe durch die Merkmale des Anspruches 1 gelöst. Weitere Aufgaben von vorteilhaften Verwendungen der Vorrichtung werden durch die Merkmale der Ansprüche 24, 25 und 29 gelöst.
   Ausgehend von einer Vorrichtung zur Positionierung eines atmenden Menschen, welcher auf einem Tisch liegt, umfasst der Tisch erfindungsgemäß einen entlang des Tisches zweidimensionalen Bereich, welcher zumindest entlang einer dem Tisch senkrechten, dritten Dimension verformbar ist und mit welchem während Atemzyklen des Menschen ein Oberkörperteil des Menschen entlang der dritten Dimension in einem im Koordinatensystem des Tisches fest gezielten Volumen positionierbar ist, als mindestens ein weiterer Oberkörperteil des Menschen durch Verformung des Bereiches frei positionierbar ist. Die Vorrichtung ist besonders vorteilhaft, um z.B. einen Tumor (oder ein Karzynom, ungesunde Zellen, etc)in einem bestimmten Volumen zu richten, in welchem eine radiotherapeutische Bestrahlung eintrifft. Dadurch können also Bewegungen des Tumors aufgrund des Atems beeinflusst werden, z.B. für eine Neuorientierung und sogar für eine Verkleinerung der Bahn des Tumors sowie also des zu strahlenden Behandlungsfelds während Atemzyklen. Komplizierte Bewegungseinrichtungen für die Bestrahlungsanlage sind daher nicht mehr erforderlich oder zumindest stark vereinfacht. Die Wahrscheinlichkeit einer 100-prozentigen Erzielung des radiotherapeutischen strahls auf dem Tumor ist damit auch wesentlich erhöht. Dies bedeutet auch, dass dank dieser Vorrichtung die Bestrahlungsdauer erheblich verkürzt werden kann, so dass der Patient mit minimierten Strahlungsmengen behandelt wird.
   Dabei kann für den verformbaren Bereich ein einfaches Kissen, verwendet werden. In diesem Fall ist die Verformung passiv. Auch weitere Ausführungen des Bereiches sind möglich, z.B. als ein oder mehrere aufblasbare Luftkissen oder als flexible Fläche mit geeignet gesteuerten Druckaktuatoren. Bei einer solchen, nun aktiven Verformung kann auch eine Steuerung der Verformung des Bereiches an dem Luftkissen oder an den Aktuatoren angeschlossen werden, welche u.a. die Bewegungsbahn eines Tumors während Atemzyklen beeinflussen kann. In diesen Sinnen kann auch als Eingangssignal das am Patienten ermittelte Atemsignal der Steuerung zugeführt werden, z.B. um die Amplitude der Tumorbahn relativ zu der Bestrahlungsanlage zu minimieren. Ausführlichere Beispiele werden im Folgenden abgegeben.
   Es können auch Einstelldaten der Verformung des Bereiches des Tisches für einen Mensch mittels am Bereich angeschlossenen Kontrollmitteln ermittelt werden oder/und diese Einstelldaten können auch in eine Planungsoberfläche für eine nachträgliche Strahlungstherapie gespeichert und aktualisiert werden.
   In der Erfindung wird vorgesehen, dass zumindest der Rücke eines Oberkörperteils des Patienten in Kontakt mit dem Tisch bzw. mit dem verformbaren Bereich ist. Die Erfindung könnte jedoch sich für einen Patient eignen, welcher nun auf dem Bauch liegt, ohne dass für einen Fachmann ein erfinderisches Zutun erforderlich wäre. Dabei sollte zumindest sein Oberköperteil in Kontakt mit dem verformbaren Bereich sein.
   Es wird auch vorgesehen, dass der Patient möglichst ruhig bleibt und also kaum in Bewegung ist. Sollte es nicht der Fall sein, ermöglicht trotzdem die Vorrichtung eine bessere Positionierungssicherheit oder -reproduzierbarkeit, da über eine oder mehrere Messeinrichtungen der Verformung der Unterlage genaue Position- oder/und Gewichtsdaten des liegenden Patienten auslösbar/geliefert werden. Ebenfalls können aus einer der Messeinrichtungen der Verformung der Unterlage Alarmsignale für eine Fehlposition des darauf liegenden Patienten auslösbar sein.
   Insbesondere werden auch mehrere vorteilhafte Verwendungen der erfindungsgemäßen Vorrichtung zur Positionierung eines atmenden, auf einem Tisch liegenden Menschen dargestellt.
   Vorteilhafte Ausgestaltungen der Erfindung sind in den Unteransprüchen dargelegt.
   Anschließend werden die Erfindung und deren Vorteile anhand der Zeichnungen erläutert.

Dabei zeigen
- FIG 1: eine Bewegung eines Tumors im Oberkörperteil des liegenden Patienten bei Vollein- und Vollausatmen,
- FIG 2: eine erfindungsgemäße Vorrichtung zur Neuorientierung der Tumorbahn zwischen Vollein- und Vollausatmen,
- FIG 3: die erfindungsgemäße Vorrichtung zur Minimierung der Tumorbahn zwischen Vollein- und Vollausatmen,
- FIG 4: ein Tisch mit einer gesteuerten, verformbaren Unterlage,
- FIG 5: eine Anlage mit der erfindungsgemäßen Vorrichtung und einer Visualisierungseinrichtung.

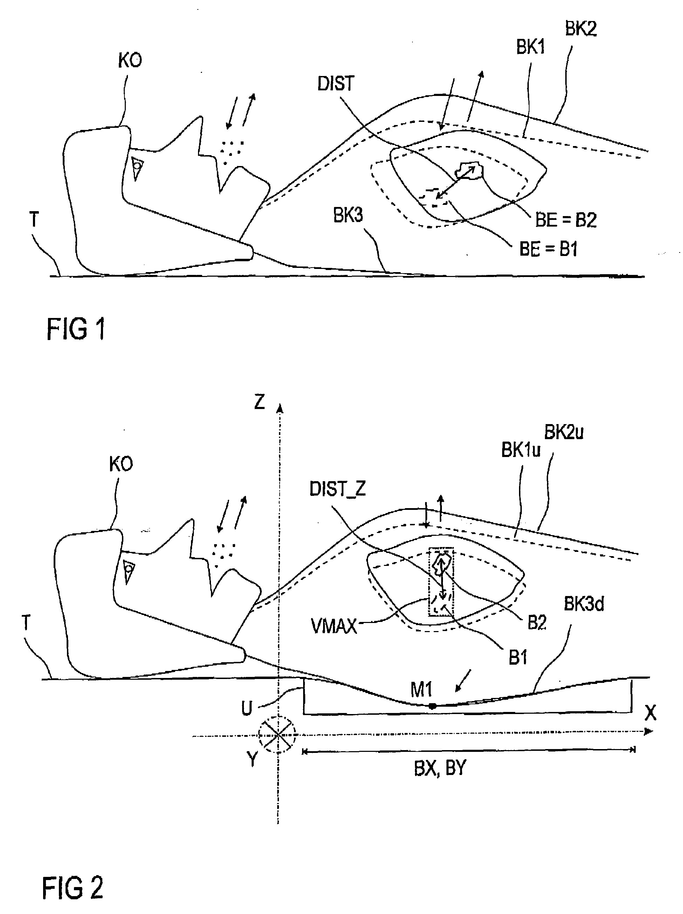

In FIG 1 ist ein auf einem Tisch T liegender Patient (Kopf KO und Rücken des Patienten sind mit dem Tisch im Kontakt) dargestellt. Dabei ist eine Bewegung eines Tumors BE in der Lunge des Patienten bzw. einer Tumorbahn zwischen zwei Positionen BE1, BE2 des Tumors und deren Entfernung DIST im Oberkörperteil des liegenden Patienten z.B. bei Vollein- und Vollausatmen gezeigt. Zu diesen beiden Positionen gehören zwei Positionen der beweglichen Oberseite BK1, BK2 des Brustkorbs des Patienten. Die Unterseite BK3 (Hinterseite des Brustkorbs, Rücken) des Patientenoberkörpers bleibt während Atemzyklen bewegungslos auf dem Tisch T, da der Tisch T eine flache, steife Platte ist. Es ist festzustellen, dass die Bahn des Tumors BE sich in allen möglichen drei-dimensionalen Raumrichtungen erstreckt. Sollte eine Bestrahlungsanlage diese Bahn verfolgen, müssen aufwendigere Mittel wie ein Roboter verwendet werden. Dazu kann ein derartiges Mittel aufgrund des nicht zu sehenden Tumors nicht garantieren, dass die Bahn der Strahlung die Bahn des Tumors treffen wird. Ein notwendiges Ein- und Ausschalten der Bestrahlungsanlage ist auch bei Erreichung des Tumors in einem davor geschätzten Referenzvolumen z.B. bei jeder Vollatmung möglich. Dadurch verlängert sich die Bestrahlungsdauer. Eine Vollatmung garantiert ferner nicht, dass der Tumor mit hoher Reproduzierbarkeit den gleichen Ort (Zielstelle der Strahlung im Oberköper) erreicht.

In Figur 2 wird die erfindungsgemäße Vorrichtung zur Neuorientierung der Tumorbahn zwischen Vollein- und Vollausatmen gemäß Figur 1 dargestellt. Im Gegensatz zu Figur 1 ist eine verformbare Unterlage U über einen entlang dem Tisch T zwei-dimensionalen Bereich BX, BY zwischen der Hinterseite BK3d und der frei beweglichen Oberseiten BKIu, BK2u des Oberkörpers des liegenden Patienten geordnet. Der verformbare Bereich BX, BY bzw. die verformbare Unterlage U kann ein unterhalb des liegenden Menschen angeordnete Kissen oder allgemeiner eine Dämpfungsfläche sein, deren Verformbarkeit von atembedingten Ausdehnungseigenschaften oder/und Gewichtseigenschaften des Oberkörpers abhängt. Durch die dreidimensionale Verformungstopographie des Kissens über den Bereich BX, BY (z.B. es wird u.a. eine Verkippung des Oberkörpers gegenüber der Y-Richtung initiiert) kann die Bahn des Tumors mit einer Länge DIST_Z während Atemzyklen vertikal (in Z-Richtung) ausgerichtet werden. D.h. in einem zum Tisch relativen Koordinatensystem X, Y, Z ist die Bahn des Tumors parallel zur vertikalen Achse Z. Damit diese korrekte Einstellung ermöglicht wird, kann während einer der Strahlungstherapie vorzeitigen Computer-Tomographie (mit welcher der Tumor und dessen Bahn visualisierbar sind) oder bei einer Aufnahme des Tumors während der Strahlungstherapie (mit z.B. einem vorgesehenen Verfahren zur Ionisierungsstrahlung) die davor unbekannte Bahn ermittelt werden und in einem Planungstherapiesystem gespeichert werden. Dabei wird rechnerisch eine Soll-Positionierung M1 der Hinterseite BK3d ermittelt, welche mit einer zwei-dimensionalen Verformungstopographie des Kissens korreliert ist, damit die bisher visualisierte Bahn bei einer anschließenden Neueinstellung der erfindungsgemäßen Vorrichtung, z.B. durch ein lokales Eindrücken des Kissens an einer Stelle M1 (Eindringspunkt, welcher eine entlang der Achse Z vertikale Vertiefung des Kissens an einer punktförmige Stelle innerhalb des Bereiches BX, BY) im Bereich BX, BY und insbesondere während der Strahlungstherapie, in eine gewünschte, vertikale Bahn umgewandelt wird. In anderen Worten bewirkt die gewünschte Verformung des Bereiches BX, BY entlang der Z-Richtung, dass während Atemzyklen des Menschen ein die Bahn umfassendes, gezielte Volumen VMAX eine zylinderförmige, möglichst geradlinige Hauptsachse aufweist, welche idealerweise zu einer der senkrechten Dreidimensionen X, Y, Z (hier die vertikale Richtung Z) im Koordinatensystem des Tisches T parallel ist. Damit wird die Einstellung des Zielorts der Bestrahlungsanlage wesentlich vereinfacht/verbessert. Eine einfache rechteckige Blende oder z.B. eine nur entlang der vertikalen Richtung T Strahlablenkungsvorrichtung für den Strahlausgang der Bestrahlungsanlage kann verwendet werden, um den Tumor zu bestrahlen. Der Komfort des Patienten ist auch noch erhöht, da ein Kissen anstelle einer steifen Platte unter ihm liegt und er steht lediglich in Kontakt mit der Unterlage U über die Hinterseite BK3d seines Brustkorbs (andere Körperteile sind frei beweglich, da kontaktfrei von allen möglichen Positionierungsmitteln). Durch ein während Atemzyklen periodisches Eindringen (und "Ausdringen") in das Kissen wird der Patient besser gehalten und daher über den Bereich BX, BY lateral genauer positioniert.

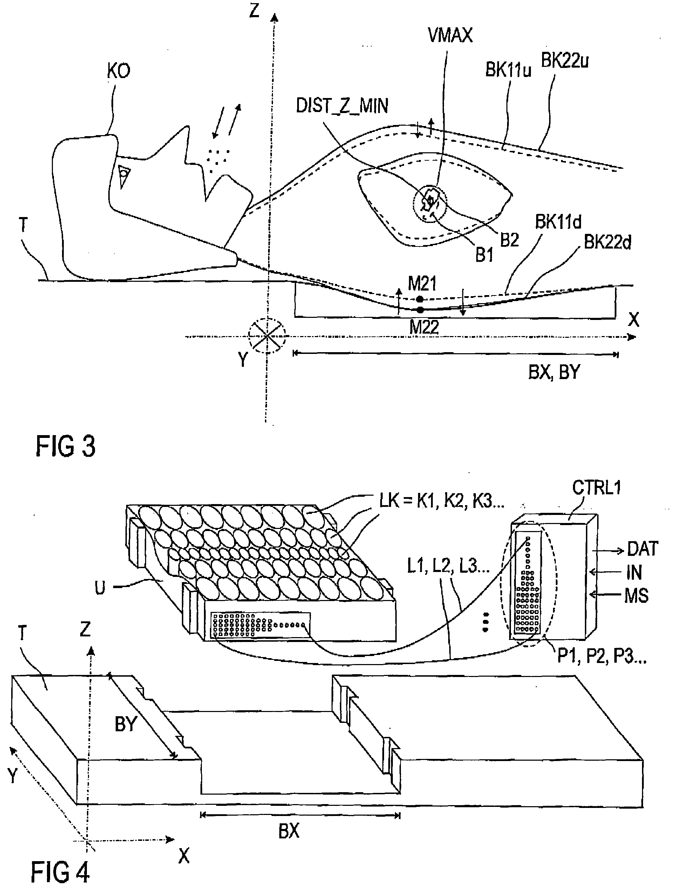

FIG 3 stellt einen zu FIG 2 weiteren Fortschritt für die erfindungsgemäße Vorrichtung dar. Nach dem gleichen Schema wird nun nicht nur die Bahn des Tumors in das bisherige zylinderförmige Volumen VMAX durch die erste Verformung des Kissens neu orientiert, aber ferner wird eine neue, atembedingte Verformung des Kissens derart angepasst, dass die Bahn oder restliche, räumliche Abweichungen des Tumors B1, 82 bzw. derer/deren zu dem Koordinatensystem X, Y, Z des Tisches T relativen Erstreckung DIST_Z_MIN zwischen Vollein- und Vollausatmen räumlich minimiert wird. Dafür wird die Verformungstopographie entlang des zwei-dimensionalen Bereiches BX, BY mittels eines oder mehrerer lokalen Eindringpunkte des Kissens bei jeder Vollatmung bzw. bei einem Atmungszyklus des Patienten z.B. während einer Therapieplanung nach einer Computer-Tomographie oder direkt mittels eines Visualisierungsverfahren bei der Strahlungstherapie ermittelt. Dabei wird wie in Figur 2 einer der Eindringspunkte für eine Soll-Positionierung M21 der positionierbaren Hinterseite BK11d des Brustkorbs erstmal derart gewählt, dass z.B. eine Neuorientierung der Bahn des Tumors gemäß Figur 2 stattfindet. Hier geschieht eine erste Allgemeinpositionierung des Patienten. Ein weiterer Eindringspunkt für eine weitere Soll-Positionierung M22 der weiter positionierbaren Hinterseite BK22d des Brustkorbs kann anschließend zur Minimierung der vertikalen Abweichungen zwischen den letzten Extrema-Positionen B1, B2 des Tumors gewählt werden. Die Hinterseite BK11d, BK22d, des Brustkorbs ist daher mittels korrekt gewählter Eindringspunkte des Kissens, und allgemeiner durch eine vertikal steuerbare Verformung über dem Bereich BX, BY, frei positionierbar. Die Höhe entlang der dritten Dimension Z (vertikal) jedes Eindringspunkts ist dementsprechend periodisch und synchron mit Atemzyklen des Patienten variierbar, so dass der Tumor an der gleichen Stelle in dem mit dem Tisch festen Koordinatensystem X, Y, Z bleibt. Es können ferner mehr als zwei Eindringspunkte verwendet werden, je nachdem was die Minimierung der räumlichen Abweichungen des Tumors erfordert. Dank dieser Methode wird es ermöglicht, der Tumor in einem im Koordinatensystem X, Y, Z des Tisches fest wählbaren Zielvolumen VMAX zu positionieren und daher die Bestrahlungsanlage andauernd, d.h. ohne zeitaufwendigen Ein- und Ausschalten während einer Strahlungstherapiephase, strahlen zu lassen. Vielmehr wird auch sicherer gestellt, dass der Tumor im Behandlungsfeld einer Bestrahlungsanlage bleibt, so dass die Bestrahlungsanlage kein aufwendigeres Positionierungsmittel mehr für ihren Strahlausgang erfordert. Vom Vorteil für den Komfort des Patienten bleibt die Oberseite BK11u, BK22u des Oberkörpers frei für eine angenehme Atmung.

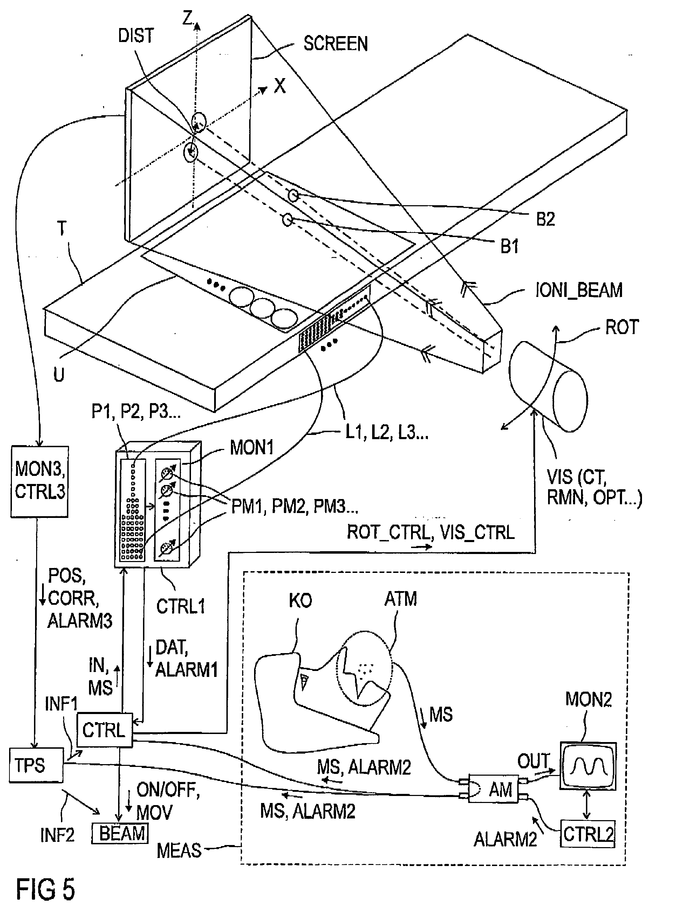

In FIG 4 wird nun der Tisch T mit einer möglichen gesteuerten, verformbaren Unterlage U dargestellt, welche in eine Vertiefung oder eine Ausnehmung des Tisches einbaubar ist, welche sich über den Bereich BX, BY erstrecken sollte und deren laterale Größe mindestens die Hinterseite des Brustkorbs des Patienten beträgt. In seiner einfachsten Form kann der verformbare Bereich BX, BY ein am Tisch fixierbares, austauschbares Kissen umfassen, welches an der Körpergröße und an dem Gewicht des Patienten angepasst wurde. Dabei kann auch die Unterlage U mindestens ein unterhalb des liegenden Menschen angeordnetes, aufblasbares Luftkissen LK = K1, K2, K3... umfassen, dessen Verformung mit mindestens einem Druckeinsteller P1, P2, P3... als erster Steuereinheit CTRL1 steuerbar ist. Als Eingangssignal weist die erste Steuereinheit CTRL1 ein Steuersignal IN beispielsweise zur Einstellung der Verformung aus einem Planungssystem oder/und ein Messsignal MS der Atemzyklen des Patienten zur zyklischen Feineinstellung der Verformung auf. Damit werden der Druck und deren atembedingte Schwankungen jeweils in einem Kissen K1, K2, K3 eingestellt. Mittels eines Ausgangssignals DAT der ersten Steuereinheit CTRL1 können zur Kontrollzwecken die Einstellungswerte des Drucks an einem Druckeinsteller P1, P2, P3,... abgegeben werden. Dafür ist ein Druckeinsteller P1, P2, P3... mit einem Druckmesser (siehe PM1, PM2, PM3... in Figur 5) verbunden. Zwischen dem Druckeinsteller P1, P2, P3... und einem Lufteingang für ein Kissen K1, K2, K3... der Unterlage U kann eine Luftleitung L1, L2, L3.... wie ein Kunststoff-Schlauch verwendet werden. Damit kann man die ganze Unterlage U mit Kissen und mit Luftleitungen L1, L2, L3..., d.h. in metall- bzw. magnetischfreien Materialen gestalten, so dass bei einer Strahlentherapie oder bei einer anderen elektromagnetisch basierten Therapie/Visualisierungsmethode die Unterlage und ihrer Verbindungen mit der ersten, von der Unterlage U entfernten Steuereinheit CTRL1 keine unerwünschte Interferenzen oder magnetische Effekte bewirken. Dieser Aspekt bildet u.a. einen wesentlichen Vorteil der Erfindung. Allgemeiner sollte der verformbare Bereich BX, BY mittels mechanischer oder/und pneumatischer Aktuatoren verformbar sein, welche idealerweise bei Anwesenheit von eines umfassenden, elektromagnetischen Feldes metall- bzw. magnetischfrei sind (z.B. in Kunststoff oder Carbon-Mikrofasern). Die erste Steuereinheit CTRL1 bzw. die Druckeinsteller P1, P2, P3...) können ferner mit weiteren Kontrollmitteln verbunden sein, welche Kontroll- bzw. Korrektursignale aus einer Computertomographie CT (über ein Therapieplanungssystem), einer Magnetresonanzabbildung, einem Verfahren zur Ionisierungsstrahlung (siehe VIS in Figur 5) oder einem anderen Visualisierung- bzw. Erkennungsmittel des Bereiches BE im Oberkörper des Menschen abgeben.

Als vorteilhafte Alternative umfasst ferner der verformbare Bereich BX, BY in Figur 4 mehrere nebeneinander angeordnete Kissen oder/und Luftkissen K1, K2, K3..., deren Verformbarkeitsgrade zumindest in der dritten Dimension Z im Koordinatensystem des Tisches (bzw. des Isozentrums für eine Bestrahlungsanlage) unterschiedlich festgelegt oder einzeln einstellbar sind. In diesem Beispiel mit mehreren nebeneinander angeordneten Luftkissen ist jeweils ein Druckeinsteller P1, P2, P3... mit einem Luftkissen K1, K2, K3... über die bereits vorgesehene Luftleitung L1, L2, L3... angeschlossen. Ebenfalls ist zur Einstellung des Drucks in einem der Luftkissen K1, K2, K3... der Druckeinsteller P1, P2, P3... mit einem oder mehreren Eingangssignalen IN, MS gesteuert, welche eine erste Grundverformung aller Kissen K1, K2, K3... und eine feinere, zyklische Verformung aller Kissen K1, K2, K3... über ein Maß einer ermittelten Atemamplitude des Menschen enthält. Damit wird der Tumor in einem eingeschränkten Volumen VMAX des Koordinatensystems X, Y, Z des Tisches T möglichst fest gehalten, trotz der atembedingten Bewegungen der Vorder-und Hinterseite des Brustkorbs.

In Figur 4 kann auch der Bereich BX, BY (d.h. der Kissen oder die Luftkissen) eine räumliche Ausnehmung wie ein Loch entlang der vertikalen Achse Z aufweisen, damit ein hoch energetischer Strahl aus einer radiotherapeutischen Bestrahlungsanlage kontaktfrei mit dem Kissen bleibt. Dies hat den Vorteil, dass z.B. die Temperatur des Kissens nicht steigt bzw. dass andere Faktoren die Einstellung des Bereiches BX, BY oder den Komfort des Patienten nicht stört. Aus Ersichtlichkeitsgründen in Figur 4 wurde diese Abnehmung nicht dargestellt.

FIG 5 stellt die erfindungsgemäßen Vorrichtung gemäß Figur 4 mit einer weiteren Visualisierungseinrichtung VIS (CT, RMN, OPT...) bei einer möglichen Bestrahlungstherapie dar. Zur Klarheit der Figur wurde eine Bestrahlungsanlage (wie ein Linearbeschleuniger) lediglich mit ihrem Strahlausgang BEAM symbolisiert und nicht dargestellt, jedoch sollte man interpretieren, dass in Betrieb die Energie (z.B. einer Gamma- oder einer Protonenstrahlung) des Strahlausgangs auf den Tumor B1, B2 konzentriert sein sollte, um ungesunde Zelle zu vernichten.

Der Druck jedes Luftkissens wird gemäß Figur 4 mittels der ersten Steuereinheit CTRL1 einmal und weiterhin dauerhaft in Zyklen eingestellt. Die Verformbarkeit der ganzen Unterlage U ist damit von atembedingten Ausdehnungseigenschaften oder/und Gewichtseigenschaften des Oberkörpers abhängig. Die Atembedingte Einstellung erfolgt mittels des ersten Eingangssignals MS, welches ein Maß einer ermittelten Atemamplitude des liegenden Menschen enthält. Zur Rückgewinnung des ersten Eingangssignal MS kann eine Druckmessvorrichtung ATM an dem Kopf KO des Patienten zur Rückgewinnung eines aus Atemwegen des Menschen aufsummierten Luftdrucks angebracht wird, aus welcher das erste Messsignal MS abgezweigt und in eine zweite Steuereinheit CTRL eingespeist wird. Die zweite Steuereinheit CTRL ist mit der ersten Steuereinheit CTRL1 verbunden ist und kann ggf. zur atembedingten, zyklischen Einstellung von Luftdruckeinstellern P1, P2, P3,... das erste Messsignal MS an die erste Steuereinheit CTRL abgegeben. Von grosser Hilfe ist es auch, dass die zweite Steuereinheit CTRL aus Informationen INF1, INF2... einer Planungsoberfläche TPS eine (Vor-)Einstellung der Verformung der Unterlage U gegenüber einer Soll-Positionierung M1, M21, M22 des Oberkörpers des Menschen steuert.

Die zweite Steuereinheit CTRL kann auch mit einer steuerbaren Monitoreinheit VIS, CT, RMN, OPT, MON3, CTRL3 verbunden sein, welche eine aktuelle Position oder/und Abweichungen zwischen aktuellen Positionen des Oberkörperteils BE, 81, B2 im Koordinatensystem X, Y, Z des Tisches T ermittelt, so dass die Soll-Positionierung M1, M21, M22 des Oberkörpers des Menschen vor einer Neueinstellung der Verformung der Unterlage aus der ersten und zweiten Steuereinheit CTRL1, CTRL neu ermittelt und ggf. aktualisiert wird. In dem Beispiel der Figur 5 könnte die Monitoreinheit anstelle der hier dargestellten Einrichtung zur Ionisierungsstrahlung VIS, einen Computertomographieanlage CT, eine auf Magnetischresonanz basierte Anlage RMN, einen optisch basiertes Abbildungssystem OPT oder/und andere Mittel zur Aufnahme eines inneren bzw. oberflächigen Teils des Oberkörpers sein, welche mittels der zweiten Steuereinheit CTRL über beispielsweise Steuersignale VIS_CTRL, ROT_CTRL zur Aufnahme und Bewegung der Monitoreinheit VIS aktivierbar sowie ggf. gegenüber dem Koordinatensystem des Tisches präzis positionierbar sind. Der Vorteil einer Einrichtung zur Ionisierungsstrahlung VIS liegt darin, dass man während einer Behandlung zur Strahlungstherapie die Einrichtung leicht am Patient anbringen kann, ohne den Patienten vom dem Tisch T zu bewegen. Damit erhöht sich die Genauigkeit bzw. Reproduzierbarkeit der Behandlung, insbesondere während einer mehrwöchigen Bestrahlung. Eine Ionisiertestrahlung VIS wird jedoch unter bestimmten Umständen durchgeführt, da in diesem Fall der Patient eine zusätzliche, ionisierte Strahlung IONI_BEAM verkraften muss, da diese aus dem divergenten Ionisierungsstrahler VIS über dem Brustkorb bis zu einer empfangsfähige Silizium-Amorph-Platte SCREEN durchgeht. Auf der Platte SCREEN werden z.B. bei Aufnahmen unter vollen An- und Ausatmung des Patienten zwei unterschiedliche Stellen des Tumors zwei-dimensional gemäß z.B. X, Z auf der die . Platte abgebildet, so dass ggf. bei Drehung ROT der Einrichtung zur Ionisierung mit dem Strahler VIS und der Platte SCREEN eine weitere Aufnahme des Tumors i.V.m. der ersten Aufnahme eine drei-dimensionale Ortbestimmung des Tumors oder/und der Bahn des Tumors ermöglicht wird. Die hiermit erhalten, räumlichen Daten, welche mittels einer Monitoreinheit MON3 erfasst werden, können dem Planungssystem TPS abgegeben, mit welchem Maßnahmen zur weiteren Steuerung bzw- zur Neueinstellung der Verformung über die Unterlage U berechnet werden.

In Figur 5 bewirkt also die erste Steuereinheit CTRL1, z.B. über Luftpumpen P1, P2, P3..., eine Verformung der Luftkissen über dem Bereich BX, BY des Tisches T gemäß Figur 4 derart, dass ein wegen Atemzyklen bewegbarer, gewählter Teil BE, B1, B2 innerhalb des Oberkörpers des Menschen, vorzugsweise ein Tumor oder ein Karzynom, oder/und mindestens eine auf dem Oberkörper des Menschen angebrachte Markierung innerhalb von dem gezielten Volumen VMAX sich erstreckt. Wie bereits erwähnt kann die erste Steuereinheit CTRL1 eine Verformung des Bereiches BX, BY derart bewirken, dass während Atemzyklen des Menschen das gezielte Volumen VMAX eine zylinderförmige, möglichst geradlinige Hauptsachse aufweist, welche idealerweise zu einer der senkrechten Dreidimensionen X, Y, Z im Koordinatensystem des Tisches parallel ist. Am besten bewirkt die erste Steuereinheit CTRL1 eine Verformung des Bereiches BX, BY derart, dass während Atemzyklen das gezielte Volumen VMAX minimale dreidimensionale Maße aufweist, idealerweise damit im Koordinatensystem X, Y, Z des Tisches der Teil BE, B1, B2 im Oberkörper des Menschen bzw. die angebrachte Markierung eine feste Lage aufweist.

Die erste Steuereinheit CTRL1 gemäß Figuren 4 und 5 weist ein Kontroll- und Kompensierungsmittel MON1 = PM1, PM2, PM3... auf, mit welchem Abweichungen (z.B. Druckabweichungen in den Luftkissen) einer eingestellten Verformung der Unterlage (U) gegenüber einer Soll-Verformung regelmäßig mittels Druckmessern PM1, PM2, PM3. ermittelt und z.B. mittels der einstellbaren Luftpumpen P1, P2, P3--. kompensiert werden.

Es können ferner Einstelldaten DAT der Verformung des Bereiches BX, BY des Tisches individuell für einen Patient mittels der Monitoreinheit MON1 = PM1, PM2, PM3... ermittelt oder/und in eine Planungsoberfläche TPS gespeichert und aktualisiert werden.

Sehr vorteilhaft sind auch nun Alarmsignale ALARM1, ALARM2, ALARM3 aus den unterschiedlichen Monitoreinheiten MON1, MON2, MON3, welche für eine ordnungsgemäße Einstellung der Verformung der Unterlage U gegenüber einer Soll-Positionierung aus einer Planungsoberfläche TPS und gegenüber atembedingten Neueinstellungen der Verformung zuständig sind, auslösbar sind. Gemäß einem Status der Alarmsignale ALARM1, ALARM2, ALARM3 kann bei einer Unsicherheit während der Strahlungstherapie die zweite Steuereinheit CTRL eine Bestrahlungsanlage BEAM rasch ein-oder ausschalten.

Nicht zu unterschätzen ist also eine Verwendung der Vorrichtung zur Positionierung eines atmenden, auf einem Tisch liegenden Menschen bei einer radio-onkologischen Behandlung im Thoraxbereich wesentlich vereinfacht, beschleunigt und sicherer durchgeführt, bei welcher ein lokaler, bewegbarer Bereich BE im Thoraxbereich eines Menschen, vorzugsweise ein Tumor oder ein Karzynom, im Visier eines vordefinierten, möglichst minimierten Strahlungsvolumens gehalten wird.

Ebenfalls ist eine Verwendung der Vorrichtung zur Positionierung eines atmenden, auf einem Tisch liegenden Menschen möglich, welche mit einem Verfahren zur Aufnahme von mindestens Schnittbildern eines Thorax, vorzugsweise einer Computertomographie CT, einer Magnetresonanzabbildung RMN, einem Verfahren VIS zur Ionisierungsstrahlung oder einem anderen visualisierungsmittel eines lokalen mit dem Thorax des atmenden Menschen bewegbaren Bereiches BE gekoppelt ist, bei welcher räumliche Abweichungen DIST des bewegbaren Bereiches BE im Thoraxbereich, vorzugsweise ein Tumor oder ein Karzynom, gegenüber einem Koordinatensystem X, Y, Z des Tisches beeinflusst oder minimiert werden.

Bei beiden Verwendungen werden zeitliche, atem- und gewichtsbedingte Formabweichungen des Thorax des Menschen in dem periodisch verformbaren Bereich BX, BY des Tisches T derart absorbiert, dass zeitliche, räumliche Abweichungen DIST des bewegbaren Bereiches BE im Thoraxbereich gegenüber dem Koordinatensystem X, Y, Z des Tisches beeinflusst oder minimiert werden. Auch können durch eine erste Voreinstellung der Verformung des Bereiches BX, BY des Tisches mindestens zwei Lokalisierungsdaten POS von entfernten Lagen B1, B2 des bewegbaren Bereiches BE im Thorax ermittelt werden, vorzugsweise bei einer Volleinatmung und bei einer Vollausatmung des Menschen, und anschließend kann mittels der Lokalisierungsdaten gegenüber einem absoluten Koordinatensystem eine Neueinstellung des verformbaren Bereiches BX, BY des Tisches mittels aktualisierter Einstelldaten IN durchgeführt werden.

Gemäß diesen Verwendungen können Werte einer räumlichen Entfernung zwischen Stellen an mindestens einer unteren bzw. oberen Oberfläche des Thorax und dem bewegbaren Bereich BE im Thorax ermittelt werden, welche in Einstelldaten für die Verformung des Bereiches BX, BY des Tisches einbezogen werden, so dass insbesondere als die untere oberflächige Stelle des Thorax mit dem verformbaren Bereich BX, BY des Tisches in Bewegung steht, die Lage des Bereiches BE im Oberkörper zumindest entlang der dritten Dimension Z im Koordinatensystem des Tisches fest bleibt.

Schließlich ist eine Verwendung der Vorrichtung zur Positionierung eines atmenden, auf einem Tisch liegenden Menschen gegeben, bei welcher zur vernünftigen jedoch für den Patient nicht anstrengenden Halterung des Thorax gegenüber dem Tisch die Verformung des Bereiches BX, BY des Tisches derart voreingestellt wird, dass eine untere Fläche des Thorax (Hinterseite, Teil des Rückens) unterhalb einer vom Tisch gebildeten Ebene ist. Damit wird der Patient in eine natürliche Weise teilweise "eingebettet". Es können auch ferner weitere Anhaltepunkte, Fixierungsoder/und Markierungsmittel zur Feinpositionierung des Menschen auf dem Tisch verwendet werden.

## Patentansprüche

1. Vorrichtung zur Positionierung eines atmenden Menschen (KO, BK1, BK2), welcher auf einem Tisch (T) liegt,
**dadurch gekennzeichnet, dass**
der Tisch einen entlang des Tisches zweidimensionalen Bereich (BX, BY) umfasst, welcher zumindest entlang einer dem Tisch senkrechten, dritten Dimension (Z) verformbar ist und mit welchem während Atemzyklen des Menschen ein Oberkörperteil (BE, B1, B2) des Menschen entlang der dritten Dimension in einem im Koordinatensystem des Tisches fest gezielten Volumen (VMAX) positionierbar ist, als mindestens ein weiterer Oberkörperteil (BK3d, BK11d, BK22d) des Menschen durch Verformung des Bereiches (BX, BY) frei positionierbar ist.

2. Vorrichtung nach Anspruch 1,
**dadurch gekennzeichnet, dass**
der verformbare Bereich (BX, BY) eine unterhalb des liegenden Menschen angeordnete Unterlage (U) ist, vorzugsweise ein Kissen, deren Verformbarkeit von atembedingten Ausdehnungseigenschaften oder/und Gewichtseigenschaften des Oberkörpers abhängt.

3. Vorrichtung nach Anspruch 2,
**dadurch gekennzeichnet, dass**
die Unterlage (U) mit einer ersten Steuereinheit (CTRL1) verbunden ist, welche ein erstes Eingangssignal (MS) umfasst,
dass das erste Eingangssignal (MS) ein Maß einer ermittelten Atemamplitude des Menschen enthält.

4. Vorrichtung nach Anspruch 3,
**dadurch gekennzeichnet, dass**
das erste Eingangssignal (MS) ein Ausgangssignal einer Einrichtung (ATM) zur Rückgewinnung eines aus Atemwegen des Menschen aufsummierten Luftdrucks ist.

5. Vorrichtung nach einem der Ansprüche 3 bis 4,
**dadurch gekennzeichnet, dass**
die erste Steuereinheit (CTRL1) mit einer zweiten Steuereinheit (CTRL) verbunden ist, welche gemäß einer Planungsoberfläche (TPS) eine Einstellung einer Verformung der Unterlage gegenüber einer Soll-Positionierung (M1, M21, M22) des Oberkörpers des Menschen steuert und welche das erste Eingangssignal (MS) empfangen kann.

6. Vorrichtung nach einem der Ansprüche 3 bis 4 und 5,
**dadurch gekennzeichnet, dass**
die zweite Steuereinheit (CTRL) mit einer steuerbaren Monitoreinheit (VIS, CT, RMN, OPT, MON3, CTRL3) verbunden ist, welche eine aktuelle Position oder/und Abweichungen zwischen aktuellen Positionen des Oberkörperteils (BE, B1, B2) im Koordinatensystem des Tisches ermittelt, so dass die Soll-Positionierung (M1, M21, M22) des Oberkörpers des Menschen vor einer Neueinstellung der Verformung der Unterlage aus der ersten und zweiten Steuereinheit (CTRL1, CTRL) neu ermittelt und ggf. aktualisiert wird.

7. Vorrichtung nach Anspruch 6,
**dadurch gekennzeichnet, dass**
die Monitoreinheit eine Einrichtung zur Ionisierungsstrahlung (VIS), einen Computertomographieanlage (CT), eine auf Magnetischresonanz basierte Anlage (PMN), einen optisch basiertes Abbildungssystem (OPT) oder/und andere Mittel zur Aufnahme eines inneren bzw. oberflächigen Teils des Oberkörpers aufweist, welche mittels der zweiten Steuereinheit (CTRL) über Steuersignale (VIS_CTRL, ROT_CTRL) aktivierbar sowie ggf. gegenüber dem Koordinatensystem des Tisches positionierbar sind.

8. Vorrichtung nach einem der Ansprüche 3 bis 7,
**dadurch gekennzeichnet, dass**
die erste Steuereinheit (CTRL1) eine Verformung des Bereiches (BX, BY) des Tisches derart bewirkt, dass ein wegen Atemzyklen bewegbarer, gewählter Teil (BE, B1, B2) innerhalb des Oberkörpers des Menschen, vorzugsweise ein Tumor oder ein Karzynom, oder/und mindestens eine auf dem Oberkörper des Menschen angebrachte Markierung innerhalb von dem gezielten Volumen (VMAX) sich erstreckt.

9. Vorrichtung nach einem der Ansprüche 3 bis 8,
**dadurch gekennzeichnet, dass**
die erste Steuereinheit (CTRL1) eine Verformung des Bereiches (BX, BY) derart bewirkt, dass während Atemzyklen des Menschen das gezielte Volumen (VMAX) eine zylinderförmige, möglichst geradlinige Hauptsachse aufweist, welche idealerweise zu einer der senkrechten Dreidimensionen (X, Y, Z) im Koordinatensystem des Tisches parallel ist.

10. Vorrichtung nach einem der Ansprüche 3 bis 9,
**dadurch gekennzeichnet, dass**
die erste Steuereinheit (CTRL1) eine Verformung des Bereiches (BX, BY) derart bewirkt, dass während Atemzyklen das gezielte Volumen (VMM) minimale dreidimensionale Maße aufweist, idealerweise damit im Koordinatensystem (X, Y, Z) des Tisches der Teil (BE, B1, B2) im Oberkörper des Menschen bzw. die angebrachte Markierung eine feste Lage aufweist.

11. Vorrichtung nach einem der Ansprüche 3 bis 10,
**dadurch gekennzeichnet, dass**
die erste Steuereinheit (CTRL1) ein Kontroll- und Kompensierungsmittel (MON1 = PM1, PM2, PM3...) aufweist, mit welchem Abweichungen einer eingestellten Verformung der Unterlage (U) gegenüber einer Soll-Verformung regelmäßig ermittelt und kompensiert werden.

12. Vorrichtung nach einem der voranstehenden Ansprüche,
**dadurch gekennzeichnet, dass**
Einstelldaten (DAT) der Verformung des Bereiches (BX, BY) des Tisches für einen Mensch mittels einer Monitoreinheit (MON1 = PM1, PM2, PM3...) ermittelt oder/und in eine Planungsoberfläche (TPS) gespeichert und aktualisiert werden.

13. Vorrichtung nach einem der voranstehenden Ansprüche 3 bis 12,
**dadurch gekennzeichnet, dass**
Alarmsignale (ALARM1, ALARM2, ALARM3) aus Monitoreinheiten (MON1, MON2, MON3), welche für eine ordnungsgemäße Einstellung der Verformung der Unterlage (U) gegenüber einer Soll-Positionierung aus einer Planungsoberfläche (TPS) und gegenüber atembedingten Neueinstellungen der Verformung zuständig sind, auslösbar sind.

14. Vorrichtung nach Anspruch 13,
**dadurch gekennzeichnet, dass**
gemäß einem Status der Alarmsignale (ALARM1, ALARM2, ALARM3) die zweite Steuereinheit (CTRL) eine Bestrahlungsanlage (BEAM) ein- oder ausschaltet.

15. Vorrichtung nach einem der voranstehenden Ansprüche,
**dadurch gekennzeichnet, dass**
der verformbare Bereich (BX, BY) mindestens ein unterhalb des liegenden Menschen angeordnetes, aufblasbares Luftkissen (K1, K2, K3...) umfasst, dessen Verformung mit mindestens einem Druckeinsteller (P1, P2, P3...) als erste Steuereinheit (CTRL1) steuerbar ist.

16. Vorrichtung nach Anspruch 15,
**dadurch gekennzeichnet, dass** der Druckeinsteller (P1, P2, P3...) mit einem Druckmesser (PM1, PM2, PM3...) verbunden ist.

17. Vorrichtung nach Anspruch 15 oder 16,
**dadurch gekennzeichnet, dass** der Druckeinsteller (P1, P2, P3...) mit Kontrollmitteln verbunden ist, welche Kontroll- bzw. Korrektursignale aus einer Computertomographie (CT), einer Magnetresonanzabbildung, einem Verfahren zur Ionisierungsstrahlung (VIS) oder einem anderen Visualisierungsmittel des Bereiches (BE) im Oberkörper des Menschen abgeben.

18. Vorrichtung nach einem der voranstehenden Ansprüche,
**dadurch gekennzeichnet, dass**
der verformbare Bereich (BX, BY) in einer Ausnehmung an einer oberen Stelle des Tisches einbaubar ist.

19. Vorrichtung nach einem der voranstehenden Ansprüche,
**dadurch gekennzeichnet, dass**
der verformbare Bereich (BX, BY) mittels mechanischer oder/und pneumatischer Aktuatoren verformbar ist, welche idealerweise bei Anwesenheit von umfassenden elektromagnetischen Feldes magnetischfrei sind.

20. Vorrichtung nach einem der voranstehenden Ansprüche,
**dadurch gekennzeichnet, dass**
der verformbare Bereich (BX, BY) ein am Tisch fixierbares, austauschbares Kissen umfasst.

21. Vorrichtung nach einem der voranstehenden Ansprüche,
**dadurch gekennzeichnet, dass**
der verformbare Bereich (BX, BY) mehrere nebeneinander angeordnete Kissen oder/und Luftkissen, deren verformbarkeitsgrade zumindest in der dritten Dimension (Z) unterschiedlich festgelegt oder einzeln einstellbar sind.

22. Vorrichtung nach Anspruch 21,
**dadurch gekennzeichnet, dass**
im Falle mehrerer nebeneinander angeordneten Luftkissen jeweils ein Druckeinsteller (P1, P2, P3...) mit einem Luftkissen (K1, K2, K3...) angeschlossen ist.

23. Vorrichtung nach Anspruch 22,
**dadurch gekennzeichnet, dass**
zur Einstellung des Drucks in einem der Luftkissen (K1, K2, K3...) der Druckeinsteller (P1, P2, P3...) mit einem Eingangssignal (IN, MS) gesteuert ist, welches ein Maß einer ermittelten Atemamplitude des Menschen enthält.

24. Verwendung der Vorrichtung zur Positionierung eines atmenden, auf einem Tisch liegenden Menschen gemäß einem der voranstehenden Ansprüche bei einer radio-onkologischen Behandlung im Thoraxbereich, bei welcher ein lokaler, bewegbarer Bereich (BE) im Thoraxbereich eines Menschen, vorzugsweise ein Tumor oder ein Karzynom, im Visier eines vordefinierten, möglichst minimierten Strahlungsvolumens gehalten wird.

25. Verwendung der Vorrichtung zur Positionierung eines atmenden, auf einem Tisch liegenden Menschen gemäß einem der voranstehenden Ansprüche 1-23, welche mit einem Verfahren zur Aufnahme von mindestens Schnittbildern eines Thorax, vorzugsweise einer Computertomographie (CT), einer Magnetresonanzabbildung, einem Verfahren (VIS) zur Ionisierungsstrahlung oder einem anderen Visualisierungsmittel eines lokalen mit dem Thorax des atmenden Menschen bewegbaren Bereiches (BE) gekoppelt ist, bei welcher räumliche Abweichungen (DIST) des bewegbaren Bereiches (BE) im Thoraxbereich, vorzugsweise ein Tumor oder ein Karzynom, gegenüber einem Koordinatensystem (X, Y, Z) des Tisches beeinflusst oder minimiert werden.

26. Verwendung nach Anspruch 24 oder 25, bei welcher zeitliche, atem- und gewichtsbedingte Formabweichungen des Thorax des Menschen in dem periodisch verformbaren Bereich (BX, BY) des Tisches (T) derart absorbiert werden, dass zeitliche, räumliche Abweichungen (DIST) des bewegbaren Bereiches (BE) im Thoraxbereich gegenüber dem Koordinatensystem (X, Y, Z) des Tisches beeinflusst oder minimiert werden.

27. Verwendung nach einem der Ansprüche 24 bis 26, bei welcher für eine erste Voreinstellung der Verformung des Bereiches (BX, BY) des Tisches mindestens zwei Lokalisierungsdaten (POS) von entfernten Lagen (B1, B2) des bewegbaren Bereiches (BE) im Thorax ermittelt werden, vorzugsweise bei einer Volleinatmung und bei einer Vollausatmung des Menschen, und anschließend mittels der Lokalisierungsdaten gegenüber einem absoluten Koordinatensystem eine Neueinstellung des verformbaren Bereiches (BX, BY) des Tisches mittels aktualisierter Einstelldaten (IN) durchgeführt wird.

28. Verwendung nach einem der Ansprüche 24 bis 27, bei welcher Werte (D1, D2, D3, D4) einer räumlichen Entfernung zwischen Stellen an mindestens einer unteren bzw. oberen Oberfläche des Thorax und dem bewegbaren Bereich (BE) im Thorax ermittelt werden, welche in Einstelldaten für die Verformung des Bereiches (BX, BY) des Tisches einbezogen werden, so dass insbesondere als die untere oberflächige Stelle des Thorax mit dem verformbaren Bereich (BX, BY) des Tisches in Bewegung steht, die Lage des Bereiches (BE) im Oberkörper zumindest entlang der dritten Dimension (Z) im Koordinatensystem des Tisches fest bleibt.

29. Verwendung der Vorrichtung zur Positionierung eines atmenden, auf einem Tisch liegenden Menschen gemäß einem der voranstehenden Ansprüche, bei welcher zur Halterung des Thorax gegenüber dem Tisch die Verformung des Bereiches (BX, BY) des Tisches derart voreingestellt wird, dass eine untere Fläche des Thorax unterhalb einer vom Tisch gebildeten Ebene ist.

30. Verwendung nach Anspruch 29, bei welcher weitere Anhaltepunkte, Fixierungs- oder/und Markierungsmittel zur Feinpositionierung des Menschen auf dem Tisch verwendet werden.
